# EUROPEAN PATENT APPLICATION

(11) **EP 4 497 456 A1**
(43) Date of publication of application: **29.01.2025**
(21) Application number: 23773429.8
(22) Date of filing: 05.01.2023
(51) Int. Cl.: A61M 5/178

(54) **PREFILLED SYRINGE WITH SHIELD, ASSEMBLY THEREOF, ASSEMBLING METHOD FOR ASSEMBLY, AND MEDICINE FILLING METHOD**

(30) Priority: 21.03.2022 CN 202210276818
(71) Applicant: Shanghai Innopac Medical Technology Co., Ltd., Shanghai 201605 (CN)
(72) Inventor: GUO, Rong, Shanghai 201605 (CN)
(74) Representative: IPrime Bonnekamp Sparing Patentanwaltsgesellschaft mbH
(86) International application number: PCT/CN2023/070627
(87) International publication number: WO 2023/179177

(57) **Abstract**

The present disclosure belongs to the technical field of medical appliances, and particularly relates to a prefilled syringe with a shield and an assembly thereof, an assembling method for the assembly, and a medicine filling method. By means of a syringe barrel, a mounting part, and a needle, as well as a shield unit sleeved on the main body of the syringe barrel, a syringe barrel flange provided on a needle mounting pipe, and a needle sleeve unit sleeved on the needle mounting pipe, deposited in the shield unit. The present disclosure has the advantages that the opening of the shield and the exposure of the needle can be achieved by means of a one-step upward pushing operation; after the shield is unlocked, the needle sleeve unit can be removed smoothly; when the shield returns to the initial position, the shield is locked with the syringe barrel flange.

## Description

### TECHNICAL FIELD

The present disclosure belongs to the technical field of medical appliances, and particularly relates to a prefilled syringe with a shield and an assembly thereof, an assembling method for the assembly, and a medicine filling method.

### BACKGROUND ART

Prefilled syringes are a novel medicine packaging form, which are primarily used for packaging and storing high-grade medicines. They are generally used for direct injection, and can also be applied to flushing procedures during surgeries. The syringes need to be discarded after being used, but needles may cause great trouble to waste recycling personnel during the discarding process, and blood remaining on the needles may cause disease transmission and harm society.

To mitigate the problem of needle exposure after discarding the prefilled syringes, a common solution is to recap previously used protective sleeves over the needles to prevent needle exposure. However, this approach has several defects: firstly, the protective sleeves, serving as temporary protectors, are prone to detachment from the needles, with an even higher likelihood of detachment occurring during the rough handling of waste transportation; secondly, the protective sleeves typically sleeve the needles snugly during the automated manufacturing process in factories, which have a very small diameter, whereas the manual method of recapping the protective sleeves over the needles is not only energy-draining and detrimental to the efficiency of nurses but also poses a high risk of needlesticking injuries to fingers of the nurses if the protective sleeves are not aligned properly with the needles during the recapping process, potentially causing the nurses to be exposed to infectious diseases from patients; and thirdly, the protective sleeves are generally designed as single-use assemblies that are often discarded after injection, making the protective sleeves difficult to locate after injection. Therefore, the method relying on recapping the protective sleeves over the needles to prevent needle exposure is unreliable.

To solve the above problem, the market has introduced shield structures specifically designed for accommodating the needles, such as the Chinese utility model patent with Publication Number CN215083511U, published on December 10, 2021, which has disclosed a detachable needle protective sleeve. This protective sleeve includes a cartridge that sleeves a needle protection cap and a protective head that engages with an end of the needle protection cap and is connected to the cartridge, and both the cartridge and the protective head are integrally formed; an engaging groove matched with the end of the needle protection cap is formed in an inner side of the protective head; and both a longitudinal section of the cartridge and a longitudinal section of the protective head are in the shape of a regular polygon, and cross-sectional areas at junctures of the cartridge and the protective head are identical. By the cartridge sleeved on the needle protection cap, any personnel using the syringe can be protected without altering the existing specifications of the syringe, and meanwhile, the protective head serves to safeguard fingers from needlesticking injuries during use.

However, this utility model further has the following defects: firstly, the detachable protective sleeve design, no matter how sophisticated the structure, inherently carries the risk of detachment from the syringe during waste transportation; and secondly, the protective head used to sleeve the needle in the utility model requires alignment with the needle for mounting, which still imposes an additional workload of nurses, with the risk of injury to the nurses.

Therefore, in order to meet market requirements, an integrated needle shield for a prefilled syringe, which is mounted with a syringe barrel during the preparation of products and can stably shield the needle from being exposed after the syringe is used, is urgently needed at present.

### SUMMARY

The present disclosure aims to provide a prefilled syringe with a shield, and an assembly thereof, an assembling method for the assembly, and a medicine filling method thereof. According to the present disclosure, a mounting part, a shield unit and a needle sleeve unit are coaxially arranged with a syringe body, the mounting part is provided with an annular flange, the shield unit includes a contractile part, a minimum inner diameter of the contractile part is smaller than a maximum outer diameter of the annular flange, the contractile part includes a first contractile part and a second contractile part, and the first contractile part and the second contractile part are respectively adjacent to a distal end and a proximal end of a maximum outer diameter position of the annular flange along an axis of the syringe body, which achieves the technical effect of stably shielding a needle after injection during use to prevent exposure.

The technical solution adopted by the present disclosure for solving the above problems is as follows: a prefilled syringe with a shield includes a syringe body, the syringe body includes a syringe barrel and a needle, the prefilled syringe further includes a mounting part, a shield unit and a needle sleeve unit, wherein the mounting part, the shield unit and the needle sleeve unit are coaxially arranged with the syringe body, one end of the mounting part is arranged at a distal end of the syringe barrel, while the other end of the mounting part is allowed for wrapping the needle, the shield unit sleeves the mounting part, the needle sleeve unit wraps the needle and at least one part of the mounting part, the mounting part is provided with an annular flange, the shield unit includes a contractile part, a minimum inner diameter of the contractile part is smaller than a maximum outer diameter of the annular flange, the contractile part includes a first contractile part and a second contractile part, the first contractile part and the second contractile part are respectively adjacent to a distal end and a proximal end of a maximum outer diameter position of the annular flange along an axis of the syringe body, an inner diameter of the first contractile part and an inner diameter of the second contractile part are allowed to be expanded, the first contractile part and the second contractile part may contract after the inner diameters are expanded, minimum expanded inner diameters of the first contractile part and the second contractile part are larger than the maximum outer diameter of the annular flange and a maximum outer diameter of the syringe barrel, and minimum contracting inner diameters of the first contractile part and the second contractile part are smaller than the maximum outer diameter of the annular flange.

In a further preferred technical solution, after the inner diameter of the first contractile part and the inner diameter of the second contractile part are expanded, the first contractile part and the second contractile part are allowed for contracting, and the minimum contracting inner diameters of the first contractile part and t the second contractile part are smaller than the maximum outer diameter of the annular flange.

In a further preferred technical solution, the needle sleeve unit, when wrapping the mounting part, is allowed for jacking up the first contractile part along a radial direction of the syringe barrel, such that the minimum inner diameter of the first contractile part can be expanded to be larger than the maximum outer diameter of the annular flange.

In a further preferred technical solution, the shield unit further includes a shield main body sleeved on the mounting part and stopping hooks which are arranged on the shield body for fitting with the proximal end of the annular flange, and the first contractile part and the second contractile part are formed at a proximal end of the shield main body.

In a further preferred technical solution, the shield unit includes cantilever members which are arranged on the shield main body and are allowed for engaging with the distal end of the annular flange.

In a further preferred technical solution, the stopping hooks and the cantilever members have a tendency to contract along the radial direction of the syringe barrel.

In a further preferred technical solution, the needle sleeve unit may be used for jacking up the cantilever members over the annular flange when the shield main body is opened.

In a further preferred technical solution, the needle sleeve unit includes a needle sleeve main body sleeved on the mounting part, and needle sleeve jack-up blocks arranged on the needle sleeve main body for jacking up the cantilever members, wherein the needle sleeve jack-up blocks may be used for jacking up the cantilever members over the annular flange when the shield main body is opened.

In a further preferred technical solution, when proximal ends of the cantilever members engage with the distal end of the annular flange, the shield main body completely wraps the needle.

In a further preferred technical solution, the syringe body further includes a frustoconical end B arranged at the distal end of the syringe barrel, and the stopping hooks engage between the frustoconical end and the annular flange when the shield unit is closed.

In a further preferred technical solution, a maximum outer diameter of the frustoconical end is larger than that of the annular flange.

In a further preferred technical solution, the contractile part further includes a third contractile part positioned at a distal end of the shield main body, and a minimum inner diameter of the third contractile part is smaller than the maximum outer diameter of the frustoconical end.

In a further preferred technical solution, the minimum inner diameter of the third contractile part is larger than the maximum outer diameter of the annular flange.

In a further preferred technical solution, the shield unit further includes a round tube limiting ring which is arranged on the shield main body and is allowed for engaging with a distal end of the frustoconical end.

An assembly of a prefilled syringe with a shield further includes a plunger rod and a plunger, wherein
the plunger rod is inserted into the plunger; and
the plunger is mounted in a syringe barrel, and is used for pushing out medicine liquid through a needle when the plunger rod is pushed toward a distal end of the syringe barrel.

An assembling method of an assembly of a prefilled syringe with a shield includes the following steps:
S 1. axially mounting a needle sleeve unit into a shield unit;
S2. sleeving the shield unit with the needle sleeve unit on a mounting part along an axial direction of a syringe barrel;
S3. pushing a plunger into the syringe barrel; and
S4. inserting the plunger rod into the plunger.

A medicine filling method of an assembly of a prefilled syringe with a shield includes the following steps:
S1. grabbing a syringe body, such that a syringe barrel is in a state of being perpendicular to a horizontal plane, and an opening at a proximal end of the syringe barrel is upward;
S2. filling a specified medicine into the syringe barrel;
S3. inserting a cartridge with a plunger into the syringe barrel, reserving a gap between an outer circumferential surface of the cartridge and an inner circumferential surface of the syringe barrel and then pushing the plunger into the syringe barrel along an inner circumferential surface of the cartridge with a cartridge plunger rod; and
S4. inserting the plunger rod into the syringe barrel, and then mounting the plunger rod on the plunger to complete a filling process.

The present disclosure has the following advantages:
Firstly, the shield and the syringe barrel are designed as an integrated unit, offering the advantage of preventing detachment. Once the shield fully shields and locks the used needle, it is nearly impossible for the needle to be exposed again, thus ensuring the effective shielding of the needle throughout the process from waste transportation until destruction.

Secondly, the shield can be opened to expose the needle through a one-step upward pushing operation, without the need for any additional unlocking steps.

Thirdly, after the shield is unlocked, the needle sleeve unit can be smoothly removed without the need to locate its position during the removal of the needle sleeve unit, thus saving both time and effort.

Fourthly, after the needle sleeve unit is removed and injection is completed, the shield returns to its initial position and is locked onto the annular flange, preventing any further movement of the shield, thus effectively shielding the needle.

Fifthly, during the locking process between the shield and the annular flange, a "click" prompt sound is generated, reminding medical staff that the needle has been successfully shielded for this syringe barrel. The syringe is allowed for disposal without the need for visual confirmation.

Sixthly, the shield does not cover a main body of the syringe barrel, ensuring that it does not interfere with visual inspection.

### BRIEF DESCRIPTION OF THE DRAWINGS

FIG. 1 is a schematic structural diagram illustrating a shield unit according to the present disclosure at a distal end when a needle sleeve unit is not removed.
FIG. 2 is a schematic structural diagram illustrating a shield unit according to the present disclosure as moved axially at a proximal end.
FIG. 3 is a schematic structural diagram illustrating that a needle sleeve unit is removed to expose a needle when a shield unit is positioned at a proximal end.
FIG. 4 is a schematic structural diagram illustrating a shield unit according to the present disclosure at a distal end after a needle sleeve unit is removed.
FIG. 5 is a structural schematic diagram illustrating a syringe barrel, a mounting part and a needle according to the present disclosure.
FIG. 6 is a schematic structural diagram illustrating a shield unit according to the present disclosure.
FIG. 7 is a schematic structural diagram illustrating a round tube limiting ring in a shield unit according to the present disclosure.
FIG. 8 is a cross-sectional view taken along a line AA of FIG. 1.
FIG. 9 is a cross-sectional view taken along a line BB of FIG. 4.

In the drawings, the reference signs denote the following components: syringe barrel A, frustoconical end B, needle C, mounting part 1, shield unit 2, needle sleeve unit 3, annular flange 101, shield main body 201, stopping hook 202, cantilever member 203, round tube limiting ring 206, needle sleeve main body 301, and needle sleeve jack-up block 302.

### DETAILED DESCRIPTION OF THE EMBODIMENTS

### Defining:

1. Distal end: one end directed toward a needle.
2. Proximal end: one end remote from the needle.
3. Axis: a straight line formed by extending infinitely along a direction of a needle.
4. Opening direction: a direction in which the needle or a needle sleeve unit can be exposed during an operation process.
5. Closing direction: a direction in which the needle or a needle sleeve unit can be wrapped during an operation process.
6. Sleeving: an act of mounting a first component on an outer surface of a second component when an inner diameter of the first component is larger than an outer diameter of the second component.
7. Arrangement: any means for combining two components together by bonding, engaging, welding, screwing, placing, or the like.
8. Jack-up: an action of applying a force by the first component onto the second component, so that the second component moves toward the direction opposite to a position of the first component.
9. Radial direction: a linear direction along a diameter or radius, or a linear direction perpendicular to an axis.

The following descriptions are only preferred embodiments of the present disclosure, but are not construed as limiting the scope of the present disclosure.

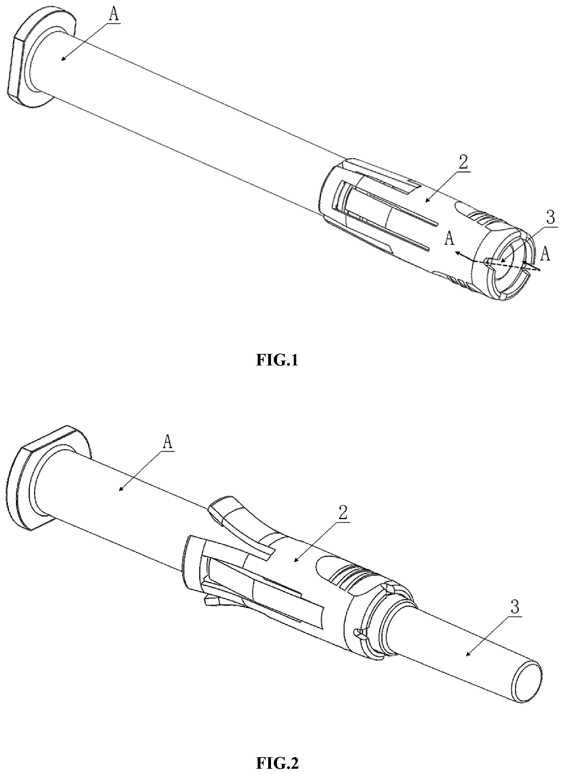

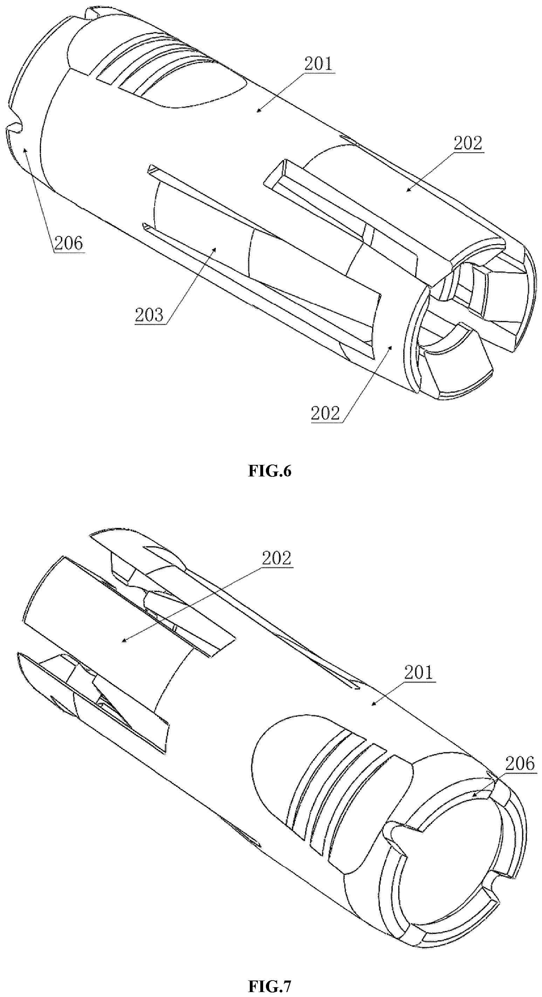

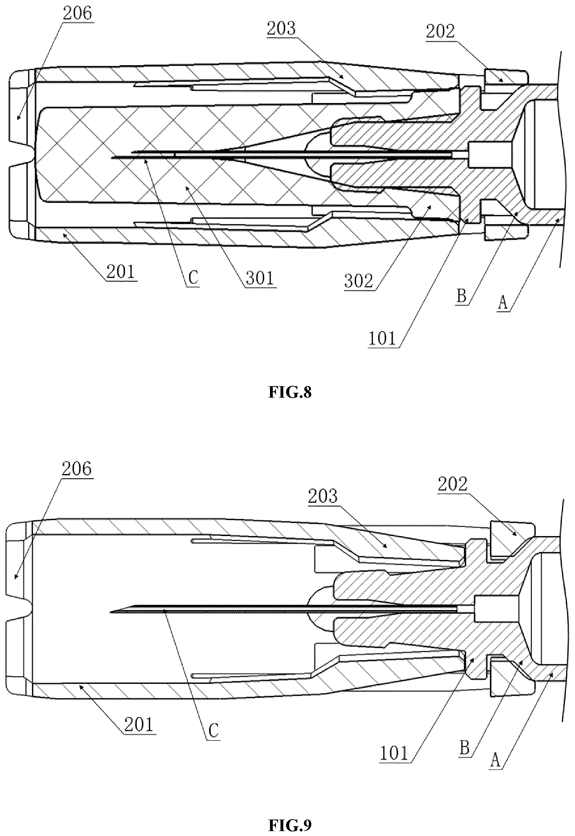

Embodiment: as shown in FIG. 1 to FIG. 9, a prefilled syringe with a shield includes a syringe body, the syringe body includes a syringe barrel A and a needle C, the prefilled syringe further includes a mounting part 1, a shield unit 2 and a needle sleeve unit 3, wherein the mounting part, the shield unit and the needle sleeve unit are coaxially arranged with the syringe body, one end of the mounting part 1 is arranged at a distal end of the syringe barrel A, while the other end of the mounting part is allowed for wrapping the needle C, the shield unit 2 sleeves the mounting part 1, the needle sleeve unit 3 wraps the needle C and at least one part of the mounting part 1, the mounting part is provided with an annular flange 101, the shield unit 2 includes a contractile part, a minimum inner diameter of the contractile part is smaller than a maximum outer diameter of the annular flange 101, the contractile part includes a first contractile part and a second contractile part, the first contractile part and the second contractile part are respectively adjacent to a distal end and a proximal end of a maximum outer diameter position of the annular flange 101 along an axis of the syringe barrel, an inner diameter of the first contractile part and an inner diameter of the second contractile part are allowed to be expanded, the first contractile part and the second contractile part may contract after the inner diameters are expanded, minimum expanded inner diameters of the first contractile part and the second contractile part are larger than the maximum outer diameter of the annular flange 101 and a maximum outer diameter of the syringe barrel A, and minimum contracting inner diameters of the first contractile part and the second contractile part are smaller than the maximum outer diameter of the annular flange 101.

In this embodiment, the syringe barrel A and the needle C are necessary structures of the existing syringe barrel, and the annular flange 101 included in the mounting part 1 is also widely present in products on the market, wherein the syringe barrel A does not include a plunger rod and a sealing rubber head for injection of the syringe barrel, such that a downstream manufacturer may conveniently inject a medicament into the syringe barrel A and then mount the plunger rod and the sealing rubber head for filling operation. In the preparation process of the products, the shield unit 2 first sleeves the needle sleeve unit 3, then the shield unit 2 and the needle sleeve unit 3 are mounted on the mounting part 1 together to form the prefilled syringe, and the prefilled syringe is sleeved with a medical sealing bag in a sterile environment. The shield unit 2 securely wraps the needle C by jointly making the first contractile part and the second contractile part engage with the annular flange 101, so as to prevent the needle from being exposed again.

Furthermore, if, in traditional methods, the needle C needs to be wrapped when discarding the syringe barrel, it is necessary to manually recap the needle sleeve unit 3 over the mounting part 1. This manual recapping process is done by aligning the needle sleeve unit 3 with a needle tip of the needle C, demanding significant attention from the nurse during the recapping process. However, when faced with a large number of injection tasks, it becomes difficult for the nurse to perform the recapping action both quickly and accurately, any slight inattention could result in accidental needlestick injuries of hands, potentially disrupting the workflow of the nurse, or in more serious cases, even exposing the nurse to infectious diseases from patients. Therefore, the significance of the shield unit 2 serving as a substitute for recapping the needle sleeve unit 3 over the needle C lies in the fact that the sleeving action, which follows the direction of the needle C, may be completed by the nurse without the need for excessive attention, and may even be completed without the nurse's gaze being focused on the syringe barrel, thus achieving a substantial significance in alleviating the workload and protecting the health of the nurse.

Furthermore, the syringe undergoes an inspection process called "visual inspection" before leaving the factory. The "visual inspection" involves visual observation of the syringe barrel to inspect whether the filling and stoppering of the syringe are up to standard. However, most integrated shields on the market affect this process. For example, the Chinese utility model with Publication No. CN214911888U, published on November 30, 2021, has disclosed a syringe needle protection device and a safety syringe, the syringe needle protection device includes an outer protective sleeve, an inner mounting sleeve, and a spring. This sleeve-in-sleeve manner of the inner sleeve and the outer sleeve obstructs the view inside the syringe barrel, resulting in impacting the visual inspection process. In contrast, the shield unit 2 in this embodiment only covers the mounting part 1 and the needle C when the syringe barrel is not in use, without shielding the syringe barrel A, as shown in FIG. 1. Therefore, the structure in this embodiment does not interfere with the visual inspection process.

In this embodiment, the general method of using the syringe barrel is as follows: firstly, the first contractile part is lifted to expand the inner diameter of the first contractile part to be larger than the maximum outer diameter of the annular flange 101, then the shield unit 2 is pushed in the opening direction, allowing the needle sleeve unit 3 to become exposed from the shield unit 2; secondly, after pushing the shield unit 2 onto the syringe barrel A, the needle sleeve unit 3 may be smoothly pulled out of the mounting part 1, exposing the needle C for completing the injection process; and thirdly, the shield unit 2 is pulled in the closing direction along the syringe barrel A, and the "closing direction" is a sliding direction along which the needle C is wrapped. When the shield unit moves to a position where the needle C is wrapped fully, the annular flange 101 seizes the shield unit 2 in place, preventing the further movement of the shield unit in the opening direction. As a result, after having been fully used, the syringe barrel may be directly discarded into a medical waste recycling apparatus for recycling, without the need for an additional operation step such as recapping the needle sleeve unit 3 over the mounting part 1.

It should be noted that the term "smoothly" in the second step indicates that the action for the nurse to unlock a protective tube and withdraw the needle sleeve unit may be completed in one seamless motion, without the need to locate the position of the needle sleeve unit 3. This design is highly beneficial in alleviating the workload of the nurse.

The needle sleeve unit 3, when wrapping the mounting part 1, is allowed for jacking up the first contractile part along a radial direction of the syringe barrel, such that the minimum inner diameter of the first contractile part is expanded to be larger than the maximum outer diameter of the annular flange 101.

In this embodiment, when the shield unit 2is sleeved on the needle sleeve unit 3, the first contractile part is jacked up by the needle sleeve unit 3, such that the shield unit 2 may directly slide along the opening direction without any unlocking operation in the presence of the needle sleeve unit 3, thus further alleviating the workload of the nurse.

Furthermore, the needle sleeve unit 3 is a necessary device of the prefilled syringe to maintain a sealing function. The first contractile part leverages the needle sleeve unit 3 necessary for the syringe barrel to serve as an unlocking device that does not engage with the annular flange 101 in the initial opening step. This remarkably ingenious design allows for the shield unit 2 to be opened without the need to deliberately lift the first contractile part for unlocking, nor manipulate any unlocking device to disengage the shield unit 2 from the annular flange 101.

Furthermore, it should be noted that the existing prefilled needle sleeve (material and shape have been standardized) is used with respect to the needle sleeve unit 3 instead of a non-standard special-shaped cap, which is advantageous in that 1. the use habit of the user is not altered; 2. the chemical compatibility of the rubber protective cap does not need to be reevaluated, thus shortening the project development cycle and product registration cycle greatly; and 3. the customer confidence is enhanced.

The shield unit 2 further includes a shield main body 201 sleeved on the mounting part 1 and stopping hooks 202 which are arranged on the shield main body 201 for fitting with the proximal end of the annular flange 101, and the first contractile part and the second contractile part are formed at a proximal end of the shield main body 201.

In this embodiment, the "sleeving" indicates that the shield main body 201 entirely wraps the syringe barrel A and may slide freely thereon. The shield main body 201 is arranged to prevent the needle C from being exposed by pulling the shield main body in the closing direction after the needle sleeve unit 3 is discarded. To ensure that the shield main body 201 does not completely detach when pulled in the closing direction, the stopping hooks 202 are arranged, which serve to stopping the movement of the shield main body 201 after the shield main body 201 completely covers the needle C, thus limiting the further movement of the shield main body 201 in the closing direction.

Additionally, one end of the stopping hook 202stopping hook 202 at a distal end tilts upward under the jack-up action of the syringe barrel A. After completing injection, the nurse may hold the syringe barrel A with one hand and push the distal end of the stopping hook 202stopping hook 202 with a finger, typically a thumb, to retract the entire shield unit 2 back over the needle C. This single-handed operation design greatly enhances the comfort level of the nurse during use.

The shield unit 2 includes cantilever members 203 which are arranged on the shield main body 201 and are allowed for engaging with the distal end of the annular flange 101.

In this embodiment, the shield unit 2 is pushed in the opening direction in the first step during the operation process, such that the needle sleeve unit 3 is exposed from the shield unit 2, the cantilever member 203 is jacked up by the needle sleeve unit 3 without engaging with the annular flange 101, such that the shield main body 201 may freely slide in the opening direction, and a friction force of the cantilever member 203 against a surface of the syringe barrel A achieves the same effect as a friction force of the stopping hooks 202 against an outer side surface of the syringe barrel A, which achieves the effect of further fixing by friction.

Furthermore, in the third step during the operation process, the needle sleeve unit 3 has been removed, the cantilever members 203 fall down at the moment when the shield main body 201 slides down and passes over the annular flange 101 and is jacked up by a side surface of the annular flange 101, meanwhile, the stopping hooks 202 are jacked up by an opposite surface of the annular flange 101, and the cantilever members 203 and the stopping hooks 202 respectively abut against both sides of the annular flange 101, such that the shield main body 201 may be securely fixed to the syringe barrel A.

The stopping hooks 202 and the cantilever members 203 have a tendency to contract along the radial direction of the syringe barrel.

In this embodiment, as shown in FIG. 2, the stopping hooks 202, when moving in the opening direction, are jacked up by the outer side surface of the syringe barrel A and bend, so as to generate the friction force against the outer side surface of the syringe barrel A. This friction force is not sufficient to limit the sliding process of the shield main body 201 on the syringe barrel A, but will limit the shield main body 201 to naturally slide toward the distal end of the shield main body in the absence of external force, for example, during the injection process of the nurse, the shield main body will not slide toward the needle C on the syringe barrel A. The significance of this design is that the operation of the nurse is not affected during the injection process.

Furthermore, due to the tendency to contract along the radial direction of the syringe barrel, the cantilever member 203 will produce a "click" prompt sound when falling down through the annular flange 101, which is intended to alert the nurse that the needle C of the syringe barrel has been completely covered and locked by the shield body 201 and may be discarded directly.

The needle sleeve unit 3 may be used for jacking up the cantilever member 203 over the annular flange 101 when the shield main body 201 is opened.

Further, the needle sleeve unit 3 includes a needle sleeve main body 301 sleeved on the mounting part 1, and needle sleeve jack-up blocks 302 arranged on the needle sleeve main body 301 for jacking up the cantilever members 203, wherein the needle sleeve jack-up blocks 302 may be used for jacking up the cantilever members 203 over the annular flange 101 when the shield main body 201 is opened.

In this embodiment, the needle sleeve jack-up blocks 302 are arranged on the needle sleeve main body 301, and are generally arranged, in an annular manner, at a distal end where the needle sleeve main body 301 sleeves the mounting part 1, and the needle sleeve jack-up blocks 302 are generally designed to fit with the side surface of the annular flange 101 on the needle sleeve main body 301, such that the cantilever members 203 may pass over the annular flange 101 without obstruction after being jacked up, and therefore, heights of the needle sleeve jack-up blocks 302are generally the same as a height of the annular flange 101 in product design. However, such a fitting-related design is not necessary, that is, gaps may also exist between the needle sleeve jack-up blocks and the annular flange, with widths being within a range that the cantilever members 203 are not obstructed from passing through freely.

Furthermore, the needle sleeve jack-up blocks 302 are of a common structure on the needle sleeve main body 301. In this embodiment, the cantilever members 203 take advantage of this structure to prevent engaging with the annular flange 101 during the initial opening step, which is an extremely ingenious design.

When proximal ends of the cantilever members 203 engage with the distal end of the annular flange 101, the shield main body 201 completely wraps the needle C.

In this embodiment, "fully wrapping" means that a maximal distal end of the shield main body 201 exceeds a maximal distal end of the needle C, which is also referred to as a fully wrapping effect.

The syringe body further includes a frustoconical end B arranged at the distal end of the syringe barrel A, and the stopping hooks 202 engage between the frustoconical end B end and the annular flange 101 when the shield unit 2 is closed.

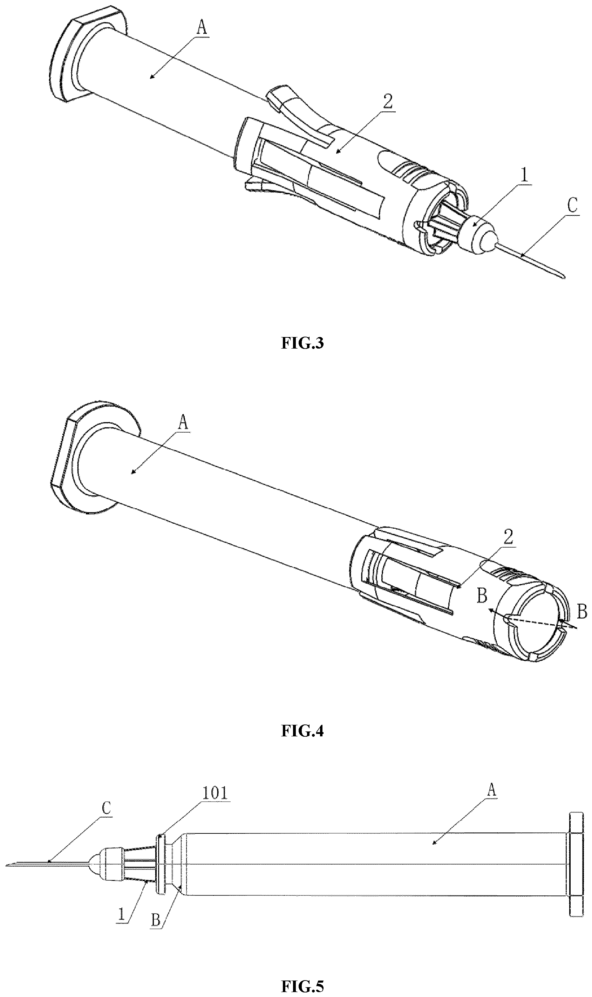

In this embodiment, a groove is formed between the frustoconical end B and the annular flange 101, as shown in FIG. 5. The groove may precisely accommodate the stopping hooks 202, allowing the stopping hooks to transition from an expanded state into a contracting state, thus reducing the volume of the syringe when it is discarded.

Furthermore, when the shield main body 201 is in the closed state, the frustoconical end B may also engage with the stopping hooks 202, such that a greater force needs to be applied to the shield main body 201 in the initial opening step, allowing the stopping hooks 202 to bend for disengaging from the frustoconical end B, thereby forming an "initial force". Under the initial force, the syringe barrel may be prevented from accidentally sliding onto the syringe barrel A to expose the needle sleeve unit 3 during transportation.

A maximum outer diameter of the frustoconical end B is larger than that of the annular flange 101.

The contractile part further includes a third contractile part positioned at a distal end of the shield main body 201, and a minimum inner diameter of the third contractile part is smaller than the maximum outer diameter of the frustoconical end B.

In this embodiment, when the minimum inner diameter of the third contractile part is smaller than the maximum outer diameter of the annular flange 101, and when the shield main body 201 is opened, a proximal end of the third contractile part is jacked up by the annular flange 101, so as to limit the shield main body 201 from continuing to move along the axial direction of the syringe barrel A.

Furthermore, when the minimum inner diameter of the third contractile part is slightly smaller than the maximum outer diameter of the annular flange 101, and when the shield main body 201 is opened, the proximal end of the third contractile part is slightly jacked up by the annular flange 101, but the third contractile part may, by slightly applying a force, be elastically expanded over the annular flange 101 and further engage between the annular flange 101 and the frustoconical end B, such that the shield main body 201 is opened to securely engage with an outer circumferential surface of the syringe barrel A.

The minimum inner diameter of the third contractile part is larger than the maximum outer diameter of the annular flange 101.

In this embodiment, when the minimum inner diameter of the third contractile part is smaller than the maximum outer diameter of the annular flange 101, and when the shield main body 201 is opened, the proximal end of the third contractile part is jacked up by the frustoconical end B, so as to limit the shield main body 201 from continuing to move along the axial direction of the syringe barrel A. Due to a higher strength of the frustoconical end B, a limiting effect achieved by the frustoconical end B is better than that achieved by the annular flange 101.

The shield unit 2 further includes a round tube limiting ring 206 which is arranged on the shield main body 201 and is allowed for engaging with a distal end of the frustoconical end B.

In this embodiment, the round tube limiting ring 206 is an expression form of the third contractile part. In order to ensure that the shield main body 201 does not move unlimitedly in the opening direction, after the needle C is completely exposed, an inner side surface of the round tube limiting ring 206 abuts against the side surface of the annular flange 101 to limit the further movement of the shield main body 201. In such a way, during an actual operation process, the nurse does not need to carefully adjust the position of the shield main body 201, but simply pushes the shield main body in the opening direction, such that the shield main body 201 may stay at a position where the needle is just exposed, and the nurse may directly push the shield main body back to an appropriate position by a thumb in the closing direction at the end of the injection.

Furthermore, the round tube limiting ring 206 has a certain deformation capability, and when a minimum inner diameter of the round tube limiting ring 206 is slightly smaller than the maximum outer diameter of the annular flange 101, with the deformation capability, the nurse may pull the shield main body 201 in the opening direction with force such that the round tube limiting ring 206 passes over the annular flange 101 to engage with the frustoconical end B, achieving a better fixing effect of the shield main body 201, and the risk of the shield main body sliding down, which may occur if the fixing of the shield main body 201 is solely reliant on the stopping hooks 202 as well as the friction force generated between the cantilever members 203 and the syringe barrel A, is avoided.

Furthermore, as shown in FIG. 3, when the round tube limiting ring 206 engages with the annular flange 101 or the frustoconical end B, a tapered head of the mounting part 1 may be fully exposed, which is very advantageous for subcutaneous injection at an angle.

An assembly of a prefilled syringe with a shield further include a plunger rod and a plunger, wherein
the plunger rod is inserted into the plunger; and
the plunger is mounted in a syringe barrel A, and is used for pushing out medicine liquid through a needle C when the plunger rod is pushed toward a distal end of the syringe barrel A.

In this embodiment, it is essential that the plunger rod is detachably connected with the plunger, such that the plunger may be easily mounted in the syringe barrel A in a mechanical and automated manner.

An assembling method for an assembly of a prefilled syringe with a shield includes the following steps:
S1. a needle sleeve unit 3 is axially mounted into a shield unit 2;
S2. the shield unit 2 with the needle sleeve unit 3 sleeves a mounting part 1 along an axial direction of a syringe barrel;
S3. a plunger is pushed into the syringe barrel A; and
S4. the plunger rod is inserted into the plunger.

In this embodiment, the needle sleeve unit 3 is mounted in the shield unit 2 first, which is beneficial to the automated assembling step of the assembly of the syringe, and may simplify the flow of the production line, reduce the cost of the assembly mounting process, and further improve the market competitiveness.

A medicine filling method of an assembly of a prefilled syringe with a shield includes the following steps:
S1. Placement of a syringe barrel: a syringe body is grabbed, such that a syringe barrel A is in a state of being perpendicular to a horizontal plane, and an opening at a proximal end of the syringe barrel is upward; and
   in this embodiment, the "grabbing" step is generally done through a mechanical arm designed by a program, and the core of the program design is to keep the syringe barrel A in a vertical state when reaching the position where a medicine is to be filled, such that a liquid level of the medicine that is filled is perpendicular to an axial direction of the syringe barrel A, thus eliminating air or leaving little air that does not interfere with the injection process after encapsulation.
S2. Medicine filling: the specified medicine is filled into the syringe barrel A; and
   in this embodiment, the "specified medicine" refers to a medicine that is self-filled according to requirements of customers, for example, for manufacturers that filling vaccines for COVID-19, the specified medicine is inactivated novel coronavirus.
S3. Stoppering for a cartridge: the cartridge with the plunger is inserted into the syringe barrel A: a gap is reserved between an outer circumferential surface of the cartridge and an inner circumferential surface of the syringe barrel A, and the plunger is pushed into the syringe barrel A along an inner circumferential surface of the cartridge with a cartridge plunger rod; and
   in this embodiment, the plunger is a device for pushing out the medicine which is commonly used in the syringe, is typically made of medical rubber, and is in a compressed state in the cartridge and expands to fit with an inner side surface of the syringe barrel A after leaving the cartridge.

Furthermore, the cartridge is an elongated steel tube and is inserted into the syringe barrel A from a crimped edge, and the plunger is advanced to a specified position with the cartridge plunger rod mounted in the cartridge, where once being withdrawn from the cartridge, the plunger is precisely positioned above the liquid level of the medicine liquid, with either no air or little air that does not interfere with the injection process between the plunger and the liquid level.

S4. Mounting of a plunger rod: the plunger rod is inserted into the syringe barrel A, and the plunger rod is then mounted on the plunger to complete a filling process.

In this embodiment, a mounting groove for mounting the plunger rod is reserved in a proximal end of the plunger, and the plunger rod is rotatably inserted into the mounting groove using a mechanical arm.

S5. The syringe that is filled undergoes visual inspection, and is labeled, and finally packaged.

The implementations of the present disclosure are described in detail above with reference to the drawings, however, the present disclosure is not limited to these. Those of ordinary skill in the art can also make various modifications within their knowledge range without departing from the purpose of the present disclosure. These modifications are not inventive, and are protected by patent laws as long as they fall within the scope of the claims of the present disclosure.

## Claims

1. A prefilled syringe with a shield, comprising a syringe body, the syringe body comprising a syringe barrel (A), a needle (C), and a needle sleeve unit (3), wherein the prefilled syringe further comprises a mounting part (1) and a shield unit (2), the mounting part and the shield unit are coaxially arranged with the syringe body, one end of the mounting part (1) is arranged at a distal end of the syringe barrel (A), while the other end of the mounting part is allowed for wrapping the needle (C), the shield unit (2) sleeves the mounting part (1), the needle sleeve unit (3) wraps the needle (C) and at least one part of the mounting part (1), the mounting part is provided with an annular flange (101), the shield unit (2) comprises a contractile part, a minimum inner diameter of the contractile part is smaller than a maximum outer diameter of the annular flange (101), the contractile part comprises a first contractile part and a second contractile part, the first contractile part and the second contractile part are respectively adjacent to a distal end and a proximal end of a maximum outer diameter position of the annular flange (101) along an axis of the syringe body, an inner diameter of the first contractile part and an inner diameter of the second contractile part are allowed to be expanded, and minimum expanded inner diameters of the first contractile part and the second contractile part are larger than the maximum outer diameter of the annular flange (101) and a maximum outer diameter of the syringe barrel (A).

2. The prefilled syringe with the shield according to claim 1, wherein after the inner diameter of the first contractile part and the inner diameter of the second contractile part are expanded, the first contractile part and the second contractile part are allowed for contracting, and minimum contracting inner diameter of the first contractile part and the second contractile part are smaller than the maximum outer diameter of the annular flange (101).

3. The prefilled syringe with the shield according to claim 1, wherein the needle sleeve unit (3), when wrapping the mounting part (1), is allowed for jacking up the first contractile part along a radial direction of the syringe barrel, such that the minimum inner diameter of the first contractile part is expanded to be larger than the maximum outer diameter of the annular flange (101).

4. The prefilled syringe with the shield according to claim 1, wherein the shield unit (2) further comprises a shield main body (201) sleeve the mounting part (1) and stopping hooks (202) which are arranged on the shield main body (201) for fitting with the proximal end of the annular flange (101), and the first contractile part and the second contractile part are formed at a proximal end of the shield main body (201).

5. The prefilled syringe with the shield according to claim 4, wherein the shield unit (2) comprises cantilever members (203) which are arranged on the shield main body (201) for engaging with the distal end of the annular flange (101).

6. The prefilled syringe with the shield according to claim 5, wherein the stopping hooks (202) and the cantilever members (203) have a tendency to contract along the radial direction of the syringe barrel.

7. The prefilled syringe with the shield according to claim 5, wherein the needle sleeve unit (3) is allowed for jacking up the cantilever members (203) over the annular flange (101) when the shield main body (201) is opened.

8. The prefilled syringe with the shield according to claim 5, wherein when proximal ends of the cantilever members (203) engage with the distal end of the annular flange (101), the shield main body (201) completely wraps the needle (C).

9. The prefilled syringe with the shield according to claim 4, wherein the syringe body further comprises a frustoconical end (B) arranged at the distal end of the syringe barrel (A), and the stopping hooks (202) engage between the frustoconical end (B) and the annular flange (101) when the shield unit (2) is closed.

10. The prefilled syringe with the shield according to claim 9, wherein a maximum outer diameter of the frustoconical end (B) is larger than the maximum outer diameter of the annular flange (101).

11. The prefilled syringe with the shield according to claim 9, wherein the contractile part further comprises a third contractile part positioned at a distal end of the shield main body (201), and a minimum inner diameter of the third contractile part is smaller than a maximum outer diameter of the frustoconical end (B).

12. The prefilled syringe with the shield according to claim 11, wherein the minimum inner diameter of the third contractile part is larger than the maximum outer diameter of the annular flange (101).

13. The prefilled syringe with the shield according to claim 9, wherein the shield unit (2) further comprises a round tube limiting ring (206) which is arranged on the shield main body (201) for engaging with a distal end of the frustoconical end (B).

14. An assembly of the prefilled syringe with the shield as claimed in any one of claims 1-13, wherein the assembly further comprise a plunger rod and a plunger, wherein
the plunger rod is inserted into the plunger; and
the plunger is mounted in a syringe barrel (A), and is allowed for pushing out medicine liquid through a needle (C) when the plunger rod is pushed toward a distal end of the syringe barrel (A).

15. An assembling method for the assembly of the prefilled syringe with the shield as claimed in claim 14, wherein the assembling method comprises the following steps:
S 1. axially mounting a needle sleeve unit (3) into a shield unit (2);
S2. sleeving the shield unit (2) with the needle sleeve unit (3) on a mounting part (1) along an axial direction of a syringe barrel;
S3. pushing a plunger into the syringe barrel (A); and
S4. inserting the plunger rod into the plunger.

16. A medicine filling method of the assembly of the prefilled syringe with the shield as claimed in claim 14, wherein the medicine filling method comprises the following steps:
S1. placing a syringe barrel: enabling the syringe barrel (A) to be in a state of being perpendicular to a horizontal plane, wherein an opening at a proximal end of the syringe barrel is upward;
S2. filling a medicine: filling a specified medicine into the syringe barrel (A);
S3. stoppering a cartridge: inserting the cartridge with the plunger into the syringe barrel (A), reserving a gap between an outer circumferential surface of the cartridge and an inner circumferential surface of the syringe barrel (A) and then pushing the plunger into the syringe barrel (A) along an inner circumferential surface of the cartridge with a cartridge plunger rod;
S4. mounting a plunger rod: inserting the plunger rod into the syringe barrel (A), and then mounting the plunger rod on the plunger.
